# EUROPEAN PATENT APPLICATION

(11) **EP 4 744 648 A1**
(43) Date of publication of application: **20.05.2026**
(21) Application number: 24825338.7
(22) Date of filing: 21.06.2024
(51) Int. Cl.: A61K 8/64, A61K 38/12, A61K 38/02, A61K 9/08, A61P 3/04

(54) **LIPOPEPTIDE-CONTAINING FAT-DISSOLVING PRODUCT, AND PREPARATION METHOD THEREFOR AND USE THEREOF**

(30) Priority: 21.06.2023 CN 202310745466
(71) Applicant: Bloomage Biotechnology Corporation Limited, Jinan, Shandong 250101 (CN)
(72) Inventor: WANG, Guanfeng, Jinan, Shandong 250101 (CN); ZHAO, Na, Jinan, Shandong 250101 (CN); MAO, Hua, Jinan, Shandong 250101 (CN); DONG, Yanmei, Jinan, Shandong 250101 (CN); GUO, Xueping, Jinan, Shandong 250101 (CN)
(74) Representative: Huang, Liwei
(86) International application number: PCT/CN2024/100671
(87) International publication number: WO 2024/260451

(57) **Abstract**

A lipopeptide-containing fat-dissolving product, and a preparation method therefor and a use thereof, relating to the field of medical aesthetics. The lipopeptide is applied to preparation of a fat-dissolving drug, and the lipopeptide can be one or more selected from surfactin, iturin, fengycin, daptomycin, polymyxin, bacillomycin L, plipastatin and bacillomycin D. The lipopeptides are directly injected into subcutaneous fatty tissues so as to reduce local adipose tissue accumulation and provide a new active component for a fat-dissolving solution. Compared with sodium deoxycholate, the lipopeptide has stronger fat-dissolving activity when being used as a fat-dissolving component, and the effective dose of the lipopeptide is lower. The lipopeptide is used as a new effective component of the fat-dissolving solution, is safer and more effective, and can reduce rupture of an injection site; and the pH of the lipopeptide is closer to the pH of a human body; therefore, the lipopeptide can relieve pain in the injection process.

## Description

### CROSS REFERENCE TO THE RELATED APPLICATIONS

This invention claims priority to Chinese Patent Application No. 202310745466.3, filed on June 21, 2023 and entitled "LIPOPEPTIDE-CONTAINING FAT-DISSOLVING PRODUCT, AND PREPARATION METHOD THEREFOR AND USE THEREOF", the entire contents of which are incorporated herein by reference.

### TECHNICAL FIELD

The present invention relates to the field of medical aesthetics, in particular to a lipopeptide-containing fat-dissolving product, and a preparation method therefor and use thereof.

### BACKGROUND

Weight loss and body contouring have always been a major area of public concern. In weight loss and body contouring methods, surgical liposuction has the most dramatic effects; however, its drawbacks involve large surgical trauma and a long recovery period. Non-surgical fat dissolution can address the drawbacks of surgical liposuction, such as high risk and prolonged recovery period, thereby improving the comfort and safety of customers. At present, the mainstream techniques of non-surgical fat dissolution include cryogenic fat dissolution, radiofrequency fat dissolution, ultrasound fat dissolution and a fat-dissolving injection. Among them, the cryogenic fat dissolution, radiofrequency fat dissolution and ultrasound fat dissolution all require the assistance of specialized equipment, while the fat-dissolving injection does not need any specialized equipment, which is a technology in which a drug is directly injected locally into the subcutaneous fat layer of the human body to dissolve adipose tissue. Currently, the fat-dissolving injection is still a scarce product. In April 2015, Kybella (active ingredient: sodium deoxycholate) developed by Allergan of the United States became the first fat-dissolving injection product approved by the U.S. Food and Drug Administration (FDA) for eliminating excess submental fat (double chin). However, no fat-dissolving injection products have yet been approved for marketing in China.

The active ingredient of the Kybella fat-dissolving injection is 1% sodium deoxycholate. Its solution also contains 0.9% benzyl alcohol, 0.44% sodium chloride, sodium hydroxide as well as inorganic salts such as phosphate, and has a pH value of 8.3. Sodium deoxycholate is a chemically synthesized small-molecule compound with the same structure as that of endogenous deoxycholic acid. Researchers have suggested that sodium deoxycholate can form precipitates if stored for a month when the pH value of its solution is below 8.2; and no precipitates are generated only when the pH value is higher than 8.2. Therefore, the pH value of the Kybella fat-dissolving injection formulation is 8.3. However, the normal physiological pH value of the human body generally ranges from 7.35 to 7.45. When the pH value and osmotic pressure of the injection formulation are different from the normal physiological values of the human body, pain will be induced.

To address the above issues, Patent CN112955125A relates to a dry powder formulation containing a deoxycholic acid component for injection, which exhibits excellent storage stability, and has the pH of 8.2 or lower. When dissolved in water for injection, the dry powder formulation forms a pH environment closer to the human physiological level than conventional injections to alleviate pain during the injection. Meanwhile, sodium deoxycholate can be fully dissolved in water for injection without the formation of precipitates.

A biological cell membrane is composed of a lipid bilayer structure. The hydrophobic chains of an amphiphilic surfactant can be inserted into a lipid bilayer membrane, so that the cell membrane is unstable and then decomposes, resulting in cell lysis. In addition, a cell membrane lipid or a cellular lysate is mixed with the surfactant to form micelles, which subsequently induces the aggregation and phagocytosis of surrounding macrophages and triggers the inflammatory reactions of the tissue. As an ionic surfactant, sodium deoxycholate exerts a fat-dissolving effect by lysing adipocytes at the injection site. Although the clinical efficacy of the Kybella fat-dissolving injection has been verified, the existing fat-dissolving injections exhibit unsatisfactory therapeutic effects and are accompanied by certain side effects according to clinical applications and market feedbacks. The common side effects of the sodium deoxycholate fat-dissolving injection include inflammatory reactions after injection, manifested as erythema and edema. Repeated inflammatory reactions caused by multiple injections may lead to fibrosis; hard nodules occasionally appear at the injection site and resolve within several weeks to months. Therefore, consumers may refrain from choosing fat-dissolving injections due to dissatisfaction with the existing therapeutic effects and fear of potential side effects. If safer and more effective fat-dissolving injections can be developed, consumer willingness to use such products will be greatly enhanced, and untapped body contouring markets will be further expanded, thereby formally ushering body contouring into the era of medical aesthetic micro-treatment.

Lipopeptides are formed by linking fatty acids to short linear or cyclic peptide chains. The structural homologs within this family differ from one another in: the type, number (7-25 residues) and configuration of amino acids in a peptide fragment; the length (12-17 carbons) and composition of a fatty acid chain, and the linking group between the two portions. The lipopeptides are generally produced metabolically by Gram-positive bacteria and exhibit a variety of biological activities. In current researches, the biological activities exhibited by the lipopeptide mainly include surfactant activity, emulsifying activity, antibacterial effect, antiviral effect, antitumor effect, hemolytic activity and anti-inflammatory activity, and can be applied in the fields of medicine, cosmetics, oil recovery, agriculture, detergents and textiles.

At present, the lipopeptides have an increasingly wide range of applications. Patent No. CN103570809B relates to an anti-inflammatory lipopeptide. The anti-inflammatory lipopeptide binds to a TLR2 receptor to induce the nuclear translocation of β-catenin. β-catenin competitively binds to PPAR-γ against p65, thereby taking an anti-inflammatory effect. Patent CN111518163A firstly proposes that a lipopeptide possesses potent anti-SARS-CoV-2 activity and can be widely applied in anti-novel coronavirus topical dermatological preparations, protective agents, detergents, personal care products and other products for inhibiting or inactivating viruses. Similarly, Patent CN111643656A proposes that a lipopeptide acts as a broad-spectrum coronavirus membrane fusion inhibitor and has an anti-human immunodeficiency virus application. The lipopeptide is a compound obtained by modifying a polypeptide with antiviral activity using a lipophilic compound. Patent CN112679576A relates to a preparation method of an antibacterial lipopeptide hydrogel and use thereof in bacteriostasis and tissue injury repair. The antibacterial lipopeptide is obtained by coupling an amino acid sequence of KKAAA(K)nAAAKK with an aliphatic hydrocarbon carboxylic acid, wherein n is an integer selected from 1 to 4. Patent CN113683673A discloses a melittin-based lipopeptide. The lipopeptide is obtained by coupling a C10-18 fatty acid to the N-terminus of melittin and performing amidation on the C-terminus of melittin. Compared with melittin, the lipopeptide exhibits better inhibitory effects against both Gram-positive and Gram-negative bacteria and has lower hemolytic activity. Patent CN113908252A discloses that surfactin can significantly inhibit Propionibacterium acnes and/or Malassezia, and relates to use of surfactin in the preparation of antimicrobial drugs and cosmetics and a preparation method thereof. It is thus evident that the applications of lipopeptides have been gradually expanding. In particular, their uses in the field of medicine are attracting increasing research attention from investigators. However, no studies have demonstrated any correlation between lipopeptides and fat dissolution.

In summary, there is an urgent need to develop a fat-dissolving injection that simultaneously has safety and fat-dissolving efficacy.

### SUMMARY

The present application aims to solve at least one of technical problems existing in the prior art or related art.

To this end, the present invention firstly adopts a lipopeptide as an active fat-dissolving substance for use in fat-dissolving drugs and fat-dissolving procedures. It is verified herein that the lipopeptide can replace the existing sodium deoxycholate component as an active fat-dissolving ingredient, thereby effectively reducing local fat accumulation while exhibiting fewer side effects than those of commercially available fat-dissolving preparations.

In one aspect, the present invention provides use of a lipopeptide in the preparation of a fat-dissolving product.

The lipopeptide includes, but is not limited to, surfactin, iturin, fengycin, daptomycin, polymyxin, bacillomycin L, plipastatin and bacillomycin D, etc.

Further, the lipopeptide is a cyclic lipopeptide; optionally, the lipopeptide is one or more selected from surfactin, iturin, fengycin, daptomycin, polymyxin, bacillomycin L, plipastatin and bacillomycin D; more optionally, the lipopeptide is surfactin and/or daptomycin; more optionally, the lipopeptide is surfactin; more optionally, the surfactin is surfactin-C14 and/or surfactin-C15, i.e., the fatty acyl moiety is a C14 and/or C15 β-hydroxy fatty acid.

The surfactin (general molecular formula: C53H93N7O13, CAS No.: 24730-31-2) is an anionic biosurfactant produced by fermentation of Bacillus subtilis, and is soluble in water and polar organic solvents. The surfactin family consists of two parts: a hydrophilic group of a cyclic heptapeptide and a β-hydroxy fatty acid chain. Its peptide ring contains 7 amino acids. The fatty acyl moiety is a C12-C16 β-hydroxy fatty acid. The carboxyl group of the amino acid at the terminus of the peptide chain is linked to the β-hydroxy group on the fatty acid chain via a lactone bond to form a ring. It has biodegradability, favorable biocompatibility and extremely low irritation, and therefore is one of highly efficient biosurfactants. Due to stable physicochemical properties, it serves as an excellent surfactant and emulsifier. Since the surfactin is formed by linking a fatty acid and a cyclic heptapeptide, its degradation products are a fatty acid and an amino acid, which are harmless to the human body. Through in vitro cell experiments and in vivo animal experiments, the present invention demonstrates that surfactin is an ideal component for eliminating local fat via injection, which exhibits a fat-dissolving effect equivalent to that of sodium deoxycholate and has fewer side effects.

Although both the lipopeptide and sodium deoxycholate are amphiphilic substances and possess hydrophilic groups and hydrophobic groups, there are significant differences between them. First, their molecular structures and molecular weights are different. The lipopeptide is composed of fatty acids and peptides, while sodium deoxycholate is a sodium cholate lacking a hydroxyl group at the C7 position. Their molecular weights are approximately 1036 Da and 414 Da, respectively. Sodium deoxycholate has a smaller molecular weight, acts on the cell membrane of adipocytes with short time, induces a relatively intense reaction, and causes a stronger inflammatory response. Furthermore, the former can be metabolized in vivo, while the latter cannot. Therefore, compared with the commonly used sodium deoxycholate fat-dissolving injections, the lipopeptide of the present invention serving as the main active ingredient for fat dissolution is safer and causes fewer side effects.

In addition, the applicant has found that sodium deoxycholate is poorly soluble when added to water, so it requires heating to be completely dissolved to form a transparent and clear fluid. After the temperature is reduced to room temperature or below, the aqueous solution of sodium deoxycholate turns into a gel state, and therefore it is needed to adjust the pH value to above 8.2 so as to avoid the gel state. Thus, the pH value of the sodium deoxycholate fat-dissolving injection is higher than 8.2. However, the normal physiological pH value of the human body generally ranges from 7.35 to 7.45, thereby causing pain upon injection. In contrast, surfactin is soluble in water, and its aqueous solution presents a transparent and clear fluid state. Therefore, the pH value of a surfactin-containing fat-dissolving injection can be close to the physiological pH value of the human body, and does not cause pain upon injection.

In an optional embodiment, the surfactin (HXRZ) is a laboratory self-prepared sample, and its preparation method refers to Patents CN114214252A and CN105695543B.

A person skilled in the art can complete the technical solution of the present invention by preparing or purchasing surfactin (surfactin-C13, surfactin-C14 and surfactin-C15). No further limitation on surfactin sources is made herein.

In an optional embodiment, surfactin-C13, surfactin-C14 and surfactin-C15 are surfactins (HXRZ) with different fatty acyl moieties purified by liquid-phase separation. A person skilled in the art can complete the separation and purification process according to actual situations and the existing technique, and therefore no specific limitation is made thereon in the present invention.

Further, the fat-dissolving comprises lysing adipocytes and/or degrading adipose tissue.

Further, the fat-dissolving drug has no rupture during the fat-dissolving process.

In another aspect, the present invention further provides a lipopeptide-containing fat-dissolving product.

Further, the lipopeptide is a cyclic lipopeptide; optionally, the lipopeptide is one or more selected from surfactin, iturin, fengycin, daptomycin, polymyxin, bacillomycin L, plipastatin and bacillomycin D; more optionally, the lipopeptide is surfactin and/or daptomycin; more optionally, the lipopeptide is surfactin; more optionally, the surfactin is surfactin-C14 and/or surfactin-C15, i.e., the fatty acyl moiety is a C14 and/or C15 β-hydroxy fatty acid.

Further, by mass percentage, the content of the lipopeptide in the fat-dissolving product is 0.05%-3%; optionally, the content of the lipopeptide in the fat-dissolving product is 0.1%-1%.

Optionally, the content of the lipopeptide in the fat-dissolving product is 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3% and any percentage therebetween.

A person skilled in the art can understand that the fat-dissolving product can also comprise a pharmaceutically acceptable solvent, carrier and/or excipient. The solvent can be one or more selected from water, normal saline and phosphate buffer saline (PBS). In addition, the fat-dissolving product can also comprise known functional ingredients such as a pH regulator, an osmotic pressure regulator, an antibacterial agent, an analgesic agent and anti-inflammatory agent to enrich the use effect of the fat-dissolving product.

As described above, it is known that sodium deoxycholate tends to form precipitates at the physiological pH of 7.35-7.45. To overcome the disadvantage, the pH value must be adjusted to 8.3, which consequently causes pain upon injection. In contrast, surfactin has no this disadvantage. Therefore, a person skilled in the art can adjust the pH of the fat-dissolving product described in the present invention as required, and no specific limitation on the pH range is set herein.

In an optional embodiment, the pH value of the fat-dissolving product ranges from 7.35 to 7.45.

Optionally, the dosage form of the product is selected from injections and/or topical formulations; optionally, the dosage form is an injection.

In another aspect, the present invention further provides a method for preparing the fat-dissolving product as described above, the method comprising: evenly dissolving a lipopeptide into a solvent.

Further, the lipopeptide is a cyclic lipopeptide; optionally, the lipopeptide is one or more selected from surfactin, iturin, fengycin, daptomycin, polymyxin, bacillomycin L, plipastatin and bacillomycin D; more optionally, the lipopeptide is surfactin and/or daptomycin; more optionally, the lipopeptide is surfactin; more optionally, the surfactin is surfactin-C14 and/or surfactin-C15, i.e., the fatty acyl moiety is a C14 and/or C15 β-hydroxy fatty acid.

More optionally, by mass percentage, the content of the lipopeptide is 0.05%-3%; more optionally, the content of the lipopeptide is 0.1%-1%.

More optionally, the content of the lipopeptide is 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3% and any percentage therebetween.

Further, the solvent is one or more selected from water, normal saline and PBS.

The present invention has the following benefits:
1. The present invention firstly discovers that the lipopeptide can be directly injected into subcutaneous adipose tissue to destroy adipocyte membranes, induce adipocyte rupture and cause local dissolution of adipose tissue, thereby achieving the purpose of fat dissolution.
2. The present invention demonstrates that, compared with sodium deoxycholate, the lipopeptide serving as the novel active ingredient of the fat-dissolving injection has higher surface activity, stronger fat-dissolving activity and lower effective dose. Through experiments related to the onset time of action, it can also be seen that the fat-dissolving mechanism of surfactin is different from that of sodium deoxycholate.

The present invention adopts the lipopeptide as the novel active ingredient for fat-dissolving injections, which can overcome the problems that commercially available sodium deoxycholate fat-dissolving injections easily cause adverse reactions and obvious pain during the use, so that the fat-dissolving injection is safer and fewer in side effects, can reduce the rupture at the injection site, and features the pH closer to the human physiological pH, thereby alleviating pain during the injection.

### BRIEF DESCRIPTION OF THE DRAWINGS

The above and/or additional aspects and advantages of the present invention will become apparent and be readily understandable from the following description of the embodiments in conjunction with the accompanying drawings, in which:
FIG. 1 is a statistical graph showing the relative growth rate of cells treated with surfactin;
FIG. 2 is a statistical graph showing the relative growth rate of cells treated with daptomycin;
FIG. 3 is a statistical graph showing the relative growth rate of cells treated with sodium deoxycholate;
FIG. 4 is a graph showing the effects of surfactin and sodium deoxycholate on mouse adipocytes (inverted microscopic observation results of adipocytes) at a magnification of 200×;
FIG. 5 is a result graph of hematoxylin-eosin stained sections of adipose tissue from the left buttock of mice at a magnification of 5×;
FIG. 6 shows fat dissolution in adipose tissue of transgenic mice treated with 0.5% surfactin (A) and 1% surfactin (B) at a magnification of 5×;
FIG. 7 shows the rupture at administration sites in mice of 2% sodium deoxycholate group and 2% surfactin group, where the circles mark the administration sites;
FIG. 8 shows the rupture at administration sites in mice of 1% sodium deoxycholate group and 1% surfactin group after two administrations, where the circles mark the administration sites;
FIG. 9 shows the degradation of inguinal adipose tissue in mice of 0.5% surfactin group, where the circles mark the administration sites;
FIG. 10 is a statistical graph showing the weight (A) and fat index (B) of left inguinal adipose tissue of mice in each group;
FIG. 11 shows representative hematoxylin-eosin (H-E) staining images of adipose tissue from mice in each group (A: negative control; B: 1% sodium deoxycholate group; C: 1% surfactin group) and statistical analysis results of the mean adipocyte density (D), with a magnification of 20×;
FIG. 12 shows the lysis effects of HXRZ, HXRZ-A, HXRZ-B and HXRZ-C on red blood cells; and
FIG. 13 shows H-E staining images of pathological sections of adipose tissue in each group (all images were captured at a magnification of 200×; A. Blank control group; B. Positive control group; C. HXRZ-B high-dose group; D. HXRZ-B medium-dose group; E. HXRZ-B low-dose group; F. HXRZ-C high-dose group; G. HXRZ-C medium-dose group; H. HXRZ-C low-dose group).

### DETAILED DESCRIPTION OF THE EMBODIMENTS

### Technical terms:

Adipose tissue: a loose connective tissue mainly composed of adipocytes. In humans, the adipose tissue is located beneath the skin (subcutaneous fat), around internal organs (visceral fat), bone marrow (yellow bone marrow), between muscles (muscular system), and breast (breast tissue).

Ulceration: generally refers to skin ulceration occurring secondary to skin conditions such as blisters, eczema and furuncles.

Topical formulation: a liquid, semi-solid or solid formulation for local action.

To more clearly illustrate the overall concept of the present invention, the present invention will be described in detail with reference to drawings of the specification by way of examples below. In the following description, numerous details are further provided to offer a thorough understanding of the present invention. However, it is obvious to a person skilled in the art that the present invention may be practiced without one or more of these details. In other examples, some technical features well known in the art are not described to avoid confusion with the present invention.

In the examples, operations without specified specific conditions shall be performed according to conventional conditions or the manufacturer's recommended conditions.

Surfactin (HXRZ) is a laboratory self-prepared sample with a purity of > 98.5%, and its preparation method refers to Patents CN114214252A and CN105695543B; surfactin HXRZ-A is surfactin-C13, a laboratory self-prepared sample obtained by liquid phase separation and purification of surfactin HXRZ; surfactin HXRZ-B is surfactin-C14, a laboratory self-prepared sample obtained by liquid phase separation and purification of surfactin HXRZ; and surfactin HXRZ-C is surfactin-C15, a laboratory self-prepared sample obtained by liquid phase separation and purification of surfactin HXRZ.

Daptomycin is purchased from Hangzhou Zhongmei Huadong Pharmaceutical Jiangdong Co., Ltd.; sodium deoxycholate is purchased from Solarbio Science & Technology Co., Ltd.; and the basal culture medium is purchased from Nanjing KeyGen Biotech Co., Ltd.

Unless otherwise specified, in the embodiments as described below, any reagents or instruments for which no manufacturer is indicated are conventional products commercially available.

### Example 1 Comparison of surface activity between surfactin and sodium deoxycholate

A 0.1% surfactin HXRZ solution and a sodium deoxycholate solution were separately prepared using purified water, followed by serial dilution to obtain different gradient concentrations. The surface tensions at different concentrations were determined respectively via a JZ-200A automatic interfacial tensiometer. The results are as shown in Table 1.

**Table 1 CMC value determination results**

| Solution concentration (%) | Surface tension value (mN/m) | |
|---|---|---|
| | Surfactin HXRZ | Sodium deoxycholate |
| 0 | 80.77 | 80.74 |
| 0.00078125 | 39.08 | 68.51 |
| 0.0015625 | 38.38 | 66.57 |
| 0.003125 | 37.64 | 65.06 |
| 0.00625 | 37.16 | 63.17 |
| 0.0125 | 36.28 | 60.50 |
| 0.025 | 35.64 | 57.94 |
| 0.05 | 33.43 | 55.40 |
| 0.1 | 33.69 | 53.47 |

It can be seen from Table 1 that 0.05% surfactin HXRZ can reduce the surface tension of water from 80 mN/m to 33 mN/m, whereas 0.1% sodium deoxycholate can only reduce the surface tension of water from 80 mN/m to 53 mN/m, indicating that the surfactin HXRZ exhibits higher surface activity than sodium deoxycholate, i.e., low-concentration surfactin HXRZ in water can readily form micelles, thereby reducing the surface tension of water. It can be speculated that when the surfactin HXRZ is injected into adipose tissue, it can act on the cytomembranes of adipocytes at a relatively low concentration to form micelles, thereby exerting a fat-dissolving effect.

### Example 2 Comparison of half maximal inhibitory concentrations (IC₅₀) of surfactin HXRZ, daptomycin and sodium deoxycholate against adipocytes

In accordance with GB/T 16886.5 Biological evaluation of medical devices-Part 5: In vitro cytotoxicity tests (GB/T 16886.5-2017/ISO 10993-5:2009, IDT), and the test was performed with reference to the methods specified therein. The detailed experimental procedures are as follows:

### 1. Seeding

Mouse adipocyte cell line 3T3-F442A in the logarithmic growth phase was seeded into a 96-well plate at a density of 1×10⁵ cells/mL, with 100 µL per well. The culture medium consisted of a basal culture medium supplemented with 10% fetal bovine serum. The seeded cells were incubated conventionally in a carbon dioxide incubator at 37°C and 5% CO2 for 24 h.

### 2. Preparation of sample solution

Surfactin HXRZ, daptomycin or sodium deoxycholate were first dissolved in PBS to prepare a 0.5% solution, and the obtained solution was sterilized by filtration through a 0.22 µm membrane filter and then diluted with a serum-free cell culture medium to a final working concentration. The solution was freshly prepared before use.

### 3. Treatment

After routine incubation for 24 h, the old culture medium was discarded, and then 100 µL of sample solution was added to replace it in the experimental group. An equal volume of serum-free culture medium was added to the normal control group. Each concentration was set up with at least three replicate wells.

### 4. Detection

After another incubation for 24 h, the relative growth rate of cells was detected by CCK-8. The culture medium was discarded, 100 µL of CCK-8 diluted 10-fold with the serum-free medium was added to each well. The plate was incubated in a cell incubator for another 2 h. The absorbance was determined using a microplate reader at a wavelength of 450 nm. The relative growth rate (RGR) is a ratio of the absorbance of the experimental group to the absorbance of the normal control group.

### 5. Results

The determination results of the relative growth rates of 3T3-F442A cells treated with surfactin HXRZ, daptomycin or sodium deoxycholate are shown in Table 2.

**Table 2 Relative growth rate (%)**

| Control group | | | 100.00±13.92 | | | | |
|---|---|---|---|---|---|---|---|
| Surfactin HXRZ | Concentration (mg/mL) | 0.005 | 0.01 | 0.02 | 0.04 | 0.08 | 0.16 |
| | Relative growth rate (%) | 101.2±15.71 | 90.23±4.30 | 67.56±3.81 | 19.34±1.43 | 10.74±0.51 | - |
| Daptomycin | Concentration (mg/mL) | 0.005 | 0.01 | 0.02 | 0.04 | 0.08 | 0.16 |
| | Relative growth rate (%) | - | 86.04±3.93 | 84. 14±2.91 | 77.87±3.87 | 74.12±1.16 | 43.11± 1.96 |
| Sodium deoxycholate | Concentration (mg/mL) | 0.125 | 0.25 | 0.3 | 0.4 | 0.5 | - |
| | Relative growth rate (%) | 98.64±1.74 | 55.58±3.66 | 23.83±3.45 | 10.92±0.48 | 10.7±0.11 | - |

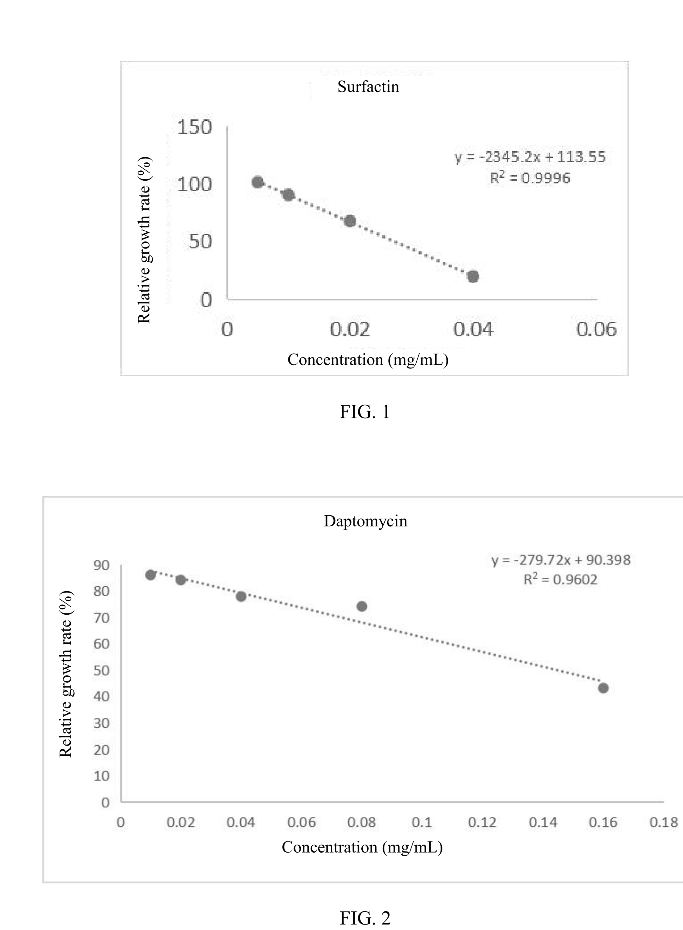

According to data in Table 2, plotting was performed by using the concentration as the abscissa and the relative growth rate of cells as the ordinate to obtain FIG. 1, FIG. 2 and FIG. 3. The half maximal inhibitory concentrations IC₅₀ of surfactin HXRZ, daptomycin and sodium deoxycholate were calculated as 0.0271 mg/mL, 0.1444 mg/mL and 0.2605 mg/mL, respectively. It can be seen that at the cellular level, both surfactin HXRZ and daptomycin have fat-dissolving activity to a certain degree, and the fat-dissolving activity of the surfactin HXRZ is superior to that of daptomycin, and the fat-dissolving activity of the daptomycin is superior to that of sodium deoxycholate.

### Example 3 Comparison of onset time of action of surfactin HXRZ and sodium deoxycholate in adipocytes

The experimental procedures in Example 3 were the same as those in Example 2 except that treatment durations were 0.5 h, 4 h and 24 h. The determination results of the relative growth rates of 3T3-F442A cells treated with surfactin HXRZ and sodium deoxycholate for different durations are shown in Table 3.

**Table 3 Relative growth rate (%)**

| | Surfactin HXRZ | | | Sodium deoxycholate | | |
|---|---|---|---|---|---|---|
| Concentration (mg/mL) | 0.08 | 0.04 | 0.02 | 0.5 | 0.4 | 0.3 |
| 0.5 h | 32.81±6.73 | 59.48±3.47 | 75.21±4.86 | 12.78±0.67 | 28.78±6.59 | 71.17±4.00 |
| 4 h | 24.38±3.37 | 37.95±1.99 | 61.07±5.11 | 17.24±0.02 | 23.81±1.24 | 61.46±9.30 |
| 24 h | 10.74±0.01 | 19.34±0.03 | 67.56±0.09 | 10.69±0.11 | 10.92±0.48 | 10.70±3.45 |

It can be seen from Table 3 that 0.5 mg/mL sodium deoxycholate completely lysed adipocytes as early as 0.5 h of treatment, while 0.08 mg/mL surfactin HXRZ completely lysed adipocytes after treatment for 24 h.

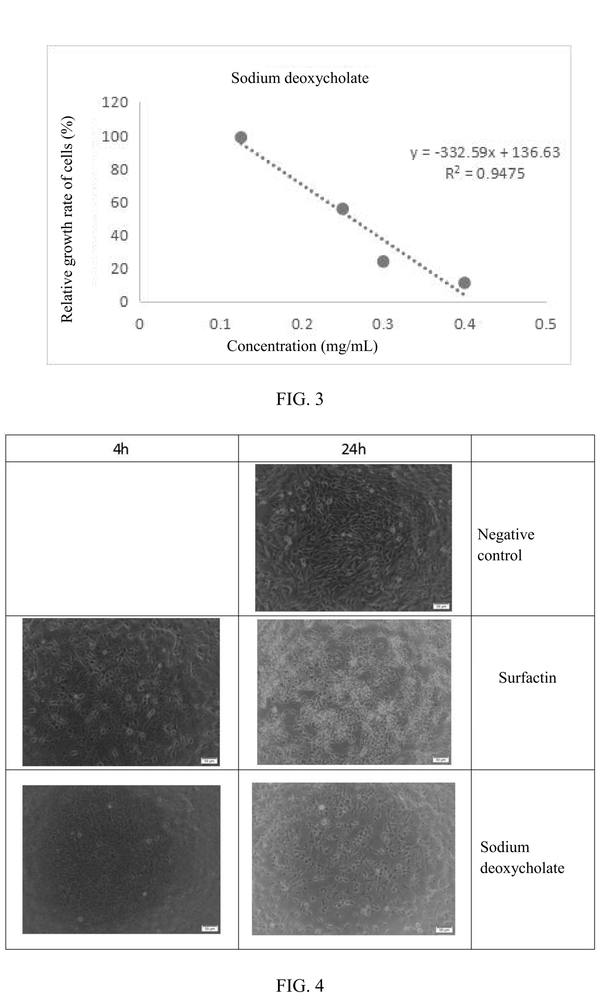

FIG. 4 shows inverted microscope images of adipocytes treated with 0.08 mg/mL surfactin HXRZ and 0.5 mg/mL sodium deoxycholate for 4 h and 24 h, respectively. It can be seen from FIG. 4 that compared with the negative control, after treatment with surfactin HXRZ for 24 h, and with sodium deoxycholate for 4 h and 24 h, almost no intact cell morphology remained, and nearly all cells died.

It can be seen that compared with sodium deoxycholate, the onset concentration of action of surfactin HXRZ in adipocytes was lower, but the onset time of action was longer. It is therefore speculated that the mechanisms of action by which surfactin HXRZ and sodium deoxycholate lyse adipocytes may be different.

### Example 4 Degradation experiment of gluteal adipose tissue in transgenic obese mice treated with surfactin HXRZ and sodium deoxycholate

According to the results in Example 2, the half maximal inhibitory concentrations IC50 of surfactin HXRZ and sodium deoxycholate were 0.0271 mg/mL and 0.2605 mg/mL, respectively. Considering that the active ingredient of the Kybella fat-dissolving injection was 1% sodium deoxycholate, the treatment concentrations of surfactin HXRZ and sodium deoxycholate in the animal experiment were therefore designed to be 0.1% and 1%, respectively.

6-week-old transgenic obese (ob/ob) mice were used as the animal model. Different drugs were injected into the adipose pads of the left buttock. The mice were totally divided into three groups with 7-10 mice per group, as specified below.
1) Negative control group: injected with 200 µL of sterile normal saline;
2) 1% sodium deoxycholate group: injected with 200 µL of sterile normal saline solution containing 1% sodium deoxycholate;
3) 0.1% surfactin HXRZ group: injected with 200 µL of sterile normal saline solution containing 0.1% surfactin HXRZ.

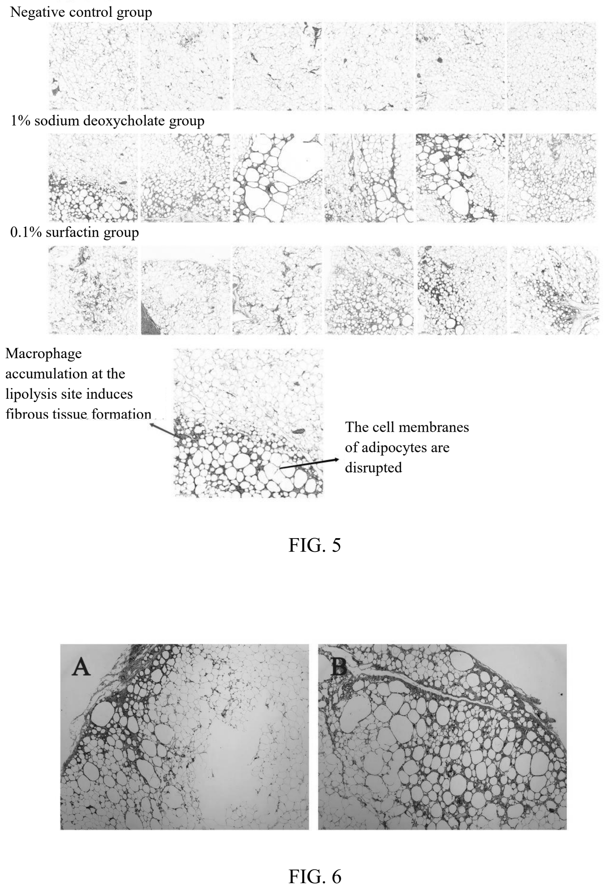

The day of the first administration was defined as Day 1 of the experiment. Additional administrations were performed on Day 3 and Day 5, respectively. The general condition and administration sites of the mice were observed regularly throughout the experiment. On Day 21 of the experiment, the mice were sacrificed and dissected. The adipose tissue of the left buttock was isolated, fixed immediately in adipose tissue fixative, and then embedded in paraffin. The adipose tissue was stained by hematoxylin and eosin staining for pathological examination, followed by observation and analysis under an optical microscope. The results are shown in FIG. 5.

As shown in FIG. 5, the observation of adipose tissue sections stained by hematoxylin and eosin staining revealed that, compared with the negative control group, both sodium deoxycholate and surfactin HXRZ induced the degradation of adipose tissue at the administration sites, with the manifestations as follows: the originally densely arranged adipocytes became irregular in morphology, with cell swelling or rupture observed; and inflammatory cell infiltration appeared in the intercellular spaces, and newly formed fibrous tissue was generated in the areas where adipocytes were ablated and cleared.

In addition, in the 1% sodium deoxycholate group, obvious induration was palpable at the administration sites in three mice, and one of the three mice developed ulceration at the administration site. No such phenomena were observed in the surfactin HXRZ group and the negative control group, indicating that no adverse reactions occurred in these two groups.

### Example 5 Degradation experiment of gluteal adipose tissue in transgenic mice treated with different concentrations of surfactin HXRZ

In this example, the degradation of gluteal adipose tissue in transgenic mice treated with different concentrations of surfactin HXRZ was further investigated.

Specifically, 6-week-old transgenic obese (ob/ob) mice were used as the animal model, with one mouse per group. 200 µL each of 0.5% and 1% surfactin HXRZ normal saline solutions (test samples) were injected into the adipose pads of the left buttock, respectively. The day of the first administration was defined as Day 1 of the experiment. Additional administrations were performed on Day 3 and Day 5, respectively. The general condition and administration sites of the mice were observed regularly throughout the experiment. On Day 21 of the experiment, the mice were sacrificed and dissected. The adipose tissue of the left buttock of the mice was isolated, fixed immediately in adipose tissue fixative, and then embedded in paraffin. The adipose tissue was stained by hematoxylin and eosin staining for pathological examination, followed by observation and analysis under an optical microscope. The results are as shown in FIG. 6.

As shown in FIG. 6, both 0.5% and 1% surfactin HXRZ induced the degradation of adipose tissue in mice. The degradation range of mouse adipose tissue in the 1% surfactin HXRZ group was greater than that in the 0.5% surfactin HXRZ group, indicating that the fat-dissolving activity of surfactin HXRZ was dose-dependent; the higher the dose, the better the fat-dissolving effect.

Example 6 Preliminary study on degradation effect of surfactin HXRZ and sodium deoxycholate on inguinal adipose tissue in mice.

6-week-old Kunming mice (weighing 20-30 g) were used as the model. A total of 200 µL of different drugs was injected subcutaneously at multiple sites with multiple needles into the left inguinal region of each mouse. The experimental groups were set as follows:
1) Negative control group (n = 3): 200 µL of sterile normal saline;
2) 1% sodium deoxycholate group (n = 3): 200 µL of 1% sodium deoxycholate normal saline solution;
3) 2% sodium deoxycholate group (n = 3): 200 µL of 2% sodium deoxycholate normal saline solution;
4) 0.5% surfactin HXRZ group (n = 3): 200 µL of 0.5% surfactin HXRZ normal saline solution;
5) 1% surfactin HXRZ group (n = 3): 200 µL of 1% surfactin HXRZ normal saline solution;
6) 2% surfactin HXRZ group (n = 3): 200 µL of 2% surfactin HXRZ normal saline solution;

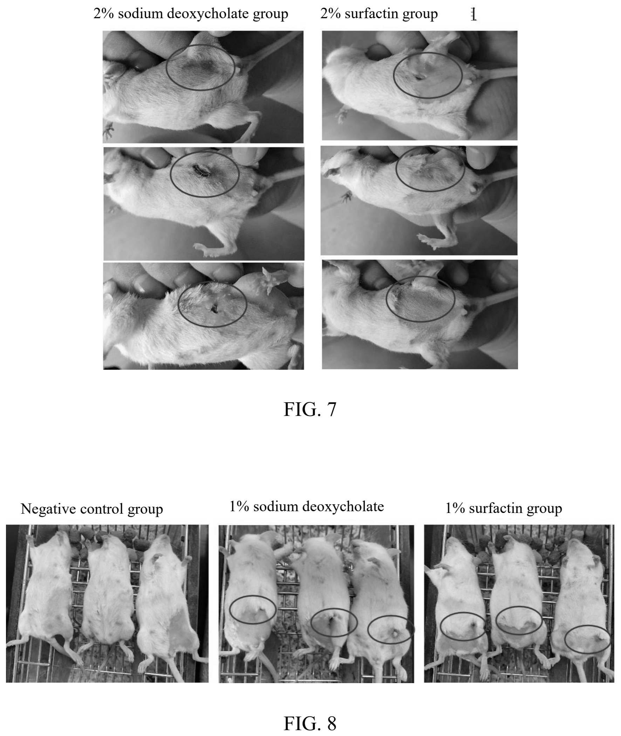

Administration was performed once every 7 days. Ulceration at the administration site of each mouse was observed throughout the administration period. On day 21 of the experiment, all mice were sacrificed and dissected. The degradation of adipose tissue in the left inguinal region was observed, as shown in FIGs. 7-9.

It can be seen from FIG. 7, after two administrations, ulceration occurred at the administration sites in all mice of the 2% sodium deoxycholate group and the 2% Surfactin HXRZ group. The ulcers failed to heal within the subsequent 7 days. Therefore, drug administration was terminated.The degree of ulceration at the administration sites in the 2% surfactin HXRZ group was milder than that in the 2% sodium deoxycholate group.

It can be seen from FIG. 8 that after two administrations, visible ulceration was observed at the administration sites in both the 1% surfactin HXRZ group and the 1% sodium deoxycholate group. The ulceration in the 1% surfactin HXRZ group was milder than that in the 1% sodium deoxycholate group. However, after a 7-day administration interval, the ulceration healed completely. After that, four additional administrations were subsequently performed, and no recurrent ulceration was observed in any group throughout the treatment period.

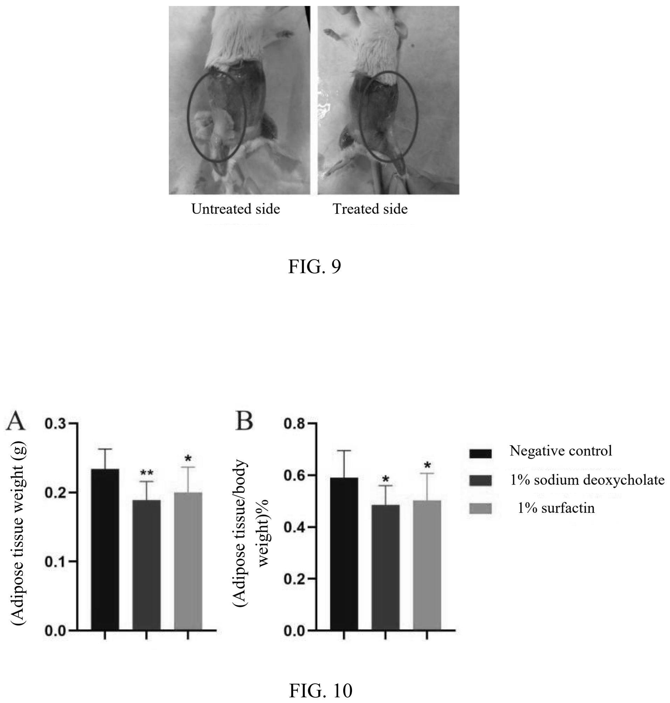

It can be seen from FIG. 9 that throughout the entire administration period, no ulceration occurred at the injection sites in mice of the 0.5% surfactin HXRZ group.After the experiment, the inguinal adipose tissue at the administration sites of the mice was dissected. It was found that compared with the untreated side tissue, the boundary of the inguinal tissue on the treated side became blurred, indicating a certain degree of degradation.

### Example 7 Study on efficacy and side effects of surfactin HXRZ and sodium deoxycholate on degradation of inguinal adipose tissue in mice

In this example, 6-week-old Kunming mice (20-30 g) were used as the model. A total of 200 µL of different drugs was injected subcutaneously at multiple sites with multiple needles into the left inguinal region of each mouse. The experimental groups were set as follows.
1) Negative control group (n = 9): 200 µL of sterile normal saline;
2) Sodium deoxycholate group (n = 9): 200 µL of 1% sodium deoxycholate sterile normal saline solution; and
3) Surfactin HXRZ group (n = 9): 200 µL of 1% surfactin HXRZ sterile normal saline solution.

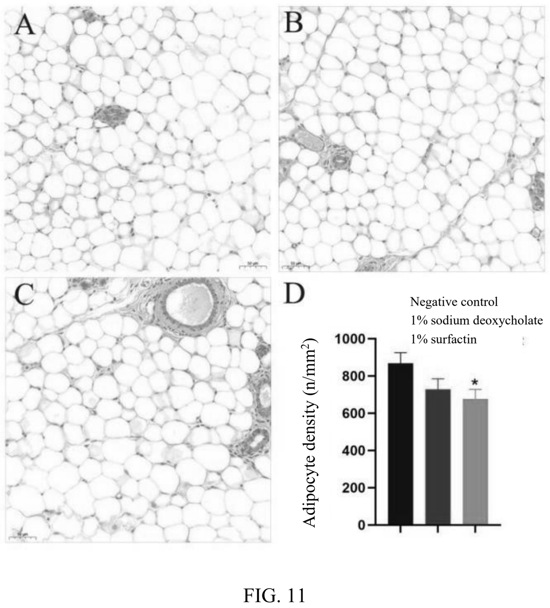

Compared with Example 6, this example adopted a modified administration interval, with the administration performed once every 14 days. On day 42 of the experiment, the mice were sacrificed and dissected. The left inguinal adipose tissue was harvested, fixed immediately in adipose tissue fixative and then embedded in paraffin. The weight and fat index of adipose tissue are shown in Table 4 and FIG. 10.The adipose tissue was stained by hematoxylin-eosin staining for pathological examination, followed by observation and analysis under an optical microscope. The adipocyte density was then calculated. The results are shown in Table 5 and FIG. 11.

**Table 4 Weight and fat index of left inguinal adipose tissue in mice of each group**

| | Adipose tissue weight (g) | Fat index (%) |
|---|---|---|
| Control group | 0.2341±0.0287 | 0.59±0.10 |
| Sodium deoxycholate group | 0.1889±0.0270** | 0.49±0.07* |
| Surfactin HXRZ group | 0.2007±0.0365* | 0.50±0.10* |

| | | |
|---|---|---|
| Note: Fat index = (Treated-side inguinal adipose weight/Body weight) × 100% *Compared with the negative control group, p<0.05; and **compared with the negative control group, p<0.01. | | |

As shown in Table 4 and FIG. 10, compared with the negative control group, subcutaneous injection of 1% surfactin HXRZ and 1% sodium deoxycholate both induced a significant reduction in mouse inguinal adipose tissue; and their efficacies were comparable with no significant statistical difference between groups.

**Table 5 Adipocyte density in mice of each group**

| | Adipocyte density (n/mm²) |
|---|---|
| Control group | 868.2±57.02 |
| Sodium deoxycholate group | 727.84±56.64* |
| Surfactin HXRZ group | 676.94±51.326* |

| | |
|---|---|
| Note: Adipocyte density = Number of adipocytes per field/Area of adipose tissue * compared with the negative control group, p<0.05. | |

It can be seen from Table 5 and FIG. 11 that by hematoxylin and eosin staining of paraffin sections of adipose tissue, whole-slide 3DHISTECH (Hungary) scanning of the sections and Media Cybernetics (U.S.A) analysis, both 1% sodium deoxycholate and 1% Surfactin HXRZ induced the degradation of adipocytes in mouse inguinal adipose tissue, thereby reducing the mean adipocyte density. As shown in FIG. 11, the mean adipocyte densities in the 1% sodium deoxycholate group and the 1% surfactin HXRZ group were 727.84±56.64 (*M̅*±SEM, the same below) and 676.94±51.326, respectively, both of which were lower than 868.2±57.02 in the negative control group. Moreover, a significant difference was observed between the 1% surfactin HXRZ group and the negative control group. It can be seen that both surfactin HXRZ and sodium deoxycholate exhibited degradation effects on the inguinal adipose tissue in mice, with no significant difference observed between the two groups. The surfactin can serve a novel active ingredient for fat dissolution.

Furthermore, in the present invention, relevant experiments were conducted to investigate the local inflammatory reactions at the administration sites induced by surfactin HXRZ and sodium deoxycholate. After administration each time, local inflammation occurred in mice of both the surfactin HXRZ group and the sodium deoxycholate group, manifesting as ulceration as well as subcutaneous nodules and induration. Statistical analysis was performed by using ulceration as an indicator of severe local inflammation and subcutaneous nodules and induration as indicators of mild local inflammation. The macrophages in pathological sections were stained by F480 immunohistochemical staining, followed by observation and analysis under an optical microscope. The detailed results are shown in Table 6.

**Table 6 Local inflammation in mice of each group**

| Administration days | Negative control group | | 1% sodium deoxycholate group | | 1% surfactin HXRZ group | |
|---|---|---|---|---|---|---|
| | Ulceration | Subcutaneous nodule and induration | Ulceration | Subcutaneous nodule and induration | Ulceration | Subcutaneous nodule and induration |
| Day 1 | 0 | 0 | 0 | 0 | 0 | 0 |
| Day 7 | 0 | 0 | 2 | 0 | 0 | 2 |
| Day 14 | 0 | 0 | 0 | 2 | 0 | 1 |
| Day 21 | 0 | 0 | 0 | 8 | 0 | 3 |
| Day 28 | 0 | 0 | 0 | 5 | 0 | 3 |
| Day 35 | 0 | 0 | 0 | 4 | 0 | 6 |
| Day 42 | 0 | 0 | 0 | 4 | 0 | 4 |

It can be seen from Table 6 that except day 35, the incidence of local inflammation in mice of the 1% surfactin HXRZ group was no higher than that in the 1% sodium deoxycholate group. Furthermore, throughout the entire administration cycle, no ulceration occurred at the administration sites in the 1% surfactin HXRZ group. In contrast, ulceration was observed at the administration sites in mice of the 1% sodium deoxycholate group after the first administration. It can be seen that compared with sodium deoxycholate, the present invention demonstrates that surfactin HXRZ serving as the fat-dissolving active ingredient has the advantages of higher safety and fewer side effects.

### Example 8 Comparison of efficacy and safety of surfactin-C13, surfactin-C14 and surfactin-C15: Toxic effects on adipocytes

In this example, the effects of fatty acid chain length and surfactin on adipocyte activity were further investigated. HXRZ, HXRZ-A, HXRZ-B and HXRZ-C were used as samples for experiment. Specifically, surfactin HXRZ-A was surfactin-C 13, surfactin HXRZ-B was surfactin-C14, and surfactin HXRZ-C was surfactin-C15.

3T3-F442A mouse adipocytes in the logarithmic growth phase were seeded into a 96-well plate at a density of 1 × 105 cells/mL, with 100 µL per well. After incubation for 24 h, the culture medium was replaced with 100 µL of culture medium containing different concentrations of samples. An equal volume of serum-free culture medium was added to the blank control group, and the incubation was continued for another 24 h. The specific concentrations are shown in Table 7. The relative growth rate of cells was determined by CCK-8. The blank control was set as 100%, the relative growth rate and half inhibitory concentration of each group were calculated. The method was the same as that in Example 2.

As shown in Table 7, the IC50 values of HXRZ, HXRZ-A, HXRZ-B and HXRZ-C against adipocytes decreased gradually. Among them, HXRZ-C exhibited the lowest IC50, which was approximately half that of HXRZ-A. That is to say, the adipocyte killing effect increased with the extension of the fatty acid carbon chain, and HXRZ-C exhibited the strongest toxicity toward adipocytes.

**Table 7 Toxicity of HXRZ and HXRZ-A, HXRZ-B and HXRZ-C to 3T3-F442A mouse adipocytes**

| Concentration (mg/mL) | Control group | HXRZ | HXRZ-A | HXRZ-B | HXRZ-C |
|---|---|---|---|---|---|
| 0.005 | | 103.67±1.59 b, c | 97.66±3.27 c | 89.75±7.04 *, #, c | 76.06±3.24 *, #, a, b |
| 0.01 | | 67.49±5.46 *, a, b, c | 97.00±3.13 #, b, c | 46.22±4.26 *, #, a | 43.53±3.35 *, #, a |
| 0.02 | 100.00±8.58 | 20.76±5.32 *, a, b, c | 34.74±3.25 *, #, b, c | 14.74±0.48 *, #, a | 17.61±1.76 *, #, a |
| 0.04 | | 9.99±0.38 *, b | 9.83±0.36 * | 9.02±0.37 *, #, c | 10.29±0.38 *, b |
| 0.08 | | 9.01±0.06 *, b, c | 9.27±0.24 *, b, c | 8.11±0.24 *, #, a, c | 10.31±0.48 *,#,a,b |

| | | | | | |
|---|---|---|---|---|---|
| Note: Values in each cell represent relative growth rate in each group (*X̅*+SD)% with the growth rate of the blank control group defined as 100%. *represents a significant difference compared with the control group (*p*< 0.05);# represents a significant difference compared with the HXRZ group at the same concentration (*p*< 0.05); a represents a significant difference compared with the HXRZ-A group at the same concentration (*p*< 0.05); b represents a significant difference compared with the HXRZ-B group at the same concentration (*p*< 0.05); and c represents a significant difference compared with the HXRZ-C group at the same concentration (*p*< 0.05). | | | | | |

### Example 9 Comparison of efficacy and safety of surfactin-C13, surfactin-C14 and surfactin-C15: Hemolytic effects on red blood cells

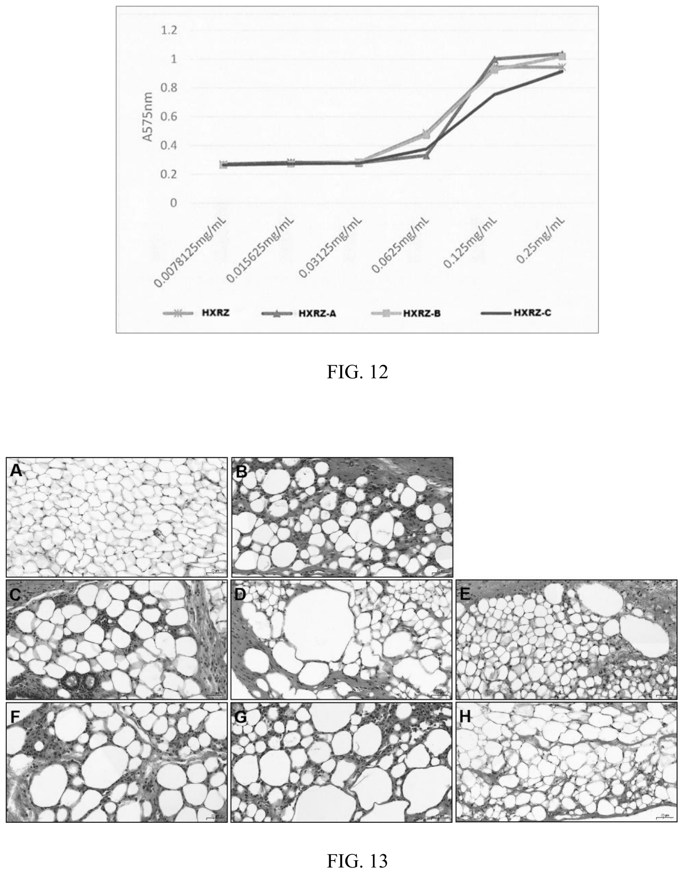

Standardized sheep red blood cells were taken and mixed with normal saline to prepare an erythrocyte suspension. Sample solutions of fermented surfactin HXRZ, HXRZ-A, HXRZ-B and HXRZ-C at different concentrations were prepared with normal saline. 1 mL of each sample solution was mixed thoroughly with 1 mL of erythrocyte suspension. An equal volume of normal saline was added to the control group. The resulting mixture was kept in a water bath at 37°C for 10 min. After centrifugation, the supernatant was collected, and the OD575 nm values were determined. The determination results and specific concentrations are shown in Table 8 and FIG. 12.

**Table 8 Hemolytic effects of HXRZ, HXRZ-A, HXRZ-B and HXRZ-C on red blood cells**

| Concentration (mg/mL) | HXRZ | HXRZ-A | HXRZ-B | HXRZ-C |
|---|---|---|---|---|
| 0.0078125 mg/mL | 0.268 | 0.2727 | 0.2657 | 0.2644 |
| 0.015625 mg/mL | 0.2834 | 0.2848 | 0.2746 | 0.2731 |
| 0.03125 mg/mL | 0.2841 | 0.2814 | 0.286 | 0.2777 |
| 0.0625 mg/mL | 0.4866 | 0.3333 | 0.4726 | 0.3761 |
| 0.125 mg/mL | 0.9445 | 1.0014 | 0.9256 | 0.7537 |
| 0.25 mg/mL | 0.9439 | 1.0377 | 1.0187 | 0.914 |

As shown in Table 8 and FIG. 12, HXRZ, HXRZ-A, HXRZ-B and HXRZ-C began to exhibit hemolytic effects on red blood cells at concentrations above 0.03125 mg/mL; almost all red blood cells were hemolyzed at 0.25 mg/mL. There was no significant difference in the hemolytic effects among HXRZ-A, HXRZ-B and HXRZ-C.

### Example 10 Comparison of efficacy and safety of surfactin-C13, surfactin-C14 and surfactin-C15: An animal experiment

### (1) Experimental scheme

Female Kunming mice (aged 7-8 weeks, weighing 25.55-33.76 g) were taken, ranked in ascending order of body weight, and divided into groups of 7-8 mice using a Latin method. The tails of mice in each cage were marked and distinguished with an oil-based permanent marker. The grouping details are as follows: 1) blank control group; 2) positive control group (10 mg/mL sodium deoxycholate); 3) HXRZ-B high-dose group (10 mg/mL); 4) HXRZ-B medium-dose group (5 mg/mL); 5) HXRZ-B low-dose group (1 mg/mL); 6) HXRZ-C high-dose group (10 mg/mL); 7) HXRZ-C medium-dose group (5 mg/mL); and 8) HXRZ-C low-dose group (1 mg/mL).

Based on the injection dose of the positive drug reported in the literature, the subcutaneous injection dose for this test was determined to be 10 mg/mL at a volume of 200 µL. Subcutaneous injection was performed using a four-needle mesotherapy tip (34 G, 1.5 mm long, inner diameter 0.6 mm). Administration was performed sequentially from top to bottom along the right inguinal region. Administration was performed once every 14 days for a total of four administrations.

During the administration period, food intake, water consumption and general activity of all experimental animals in each group were observed daily under identical feeding conditions and housing environments. Daily observation was performed on each mouse to check for ulceration, induration and other lesions at the administration site. 14 days after the second administration, two mice from each group were sacrificed and dissected. All the remaining mice were sacrificed and dissected 14 days after the fourth administration.

### (2) Experimental results

### 2.1 Local adverse reaction

**In** this example, local adverse reactions at the administration sites of the experimental mice were statistically analyzed, and the statistical results are shown in Table 9. Except for adverse reactions occurring after the first administration in the HXRZ-B high-dose group, the incidence of adverse reactions in the positive control group exceeded 60%, and the incidence of adverse reactions in the sample groups was lower than 60%.

**Table 9 Incidence of local adverse reactions in mice of each group after administration**

| Group | First administration | Second administration | Third administration | Fourth administration |
|---|---|---|---|---|
| Blank control group | 0.00 | 0.00 | 0.00 | 0.00 |
| Positive control group | 62.50 | 75.00 | 66.67 | 83.33 |
| HXRZ-B high-dose group | 100.00 | 28.57 | 60.00 | 40.00 |
| HXRZ-B medium-dose group | 14.29 | 57.14 | 0.00 | 0.00 |
| HXRZ-B low-dose group | 0.00 | 0.00 | 0.00 | 0.00 |
| HXRZ-C high-dose group | 42.86 | 28.57 | 20.00 | 40.00 |
| HXRZ-C medium-dose group | 14.29 | 28.57 | 0.00 | 20.00 |
| HXRZ-C low-dose group | 0.00 | 14.29 | 0.00 | 0.00 |

| | | | | |
|---|---|---|---|---|
| Note: the values in each cell represent the percentage (%) of mice with abnormalities at the administration site. Administration site abnormalities refer to erythema and swelling, dark red scabbing and skin ulceration. Before the third administration, two mice from each group were dissected to observe the staged treatment outcomes, which accordingly reduced the number of animals in each group. | | | | |

### 2.2 Adipose tissue changes at administration site

**In** this example, changes in adipose tissue at the administration sites of experimental mice were statistically analyzed. Specifically, the adipose tissue was excised from the inguinal region on the inoculated side (right side), weighed and recorded. The adipose tissue-to-body weight percentage of the inoculated side for each mouse was then calculated using the formula: [(weight of adipose tissue on the inoculated side/body weight of the mouse) × 100%]. The experimental results are shown in Table 10. The mean wet adipose tissue weight and wet adipose tissue-to-body weight percentage of mice in the positive drug group and each administration group were both lower than those in the blank control group, indicating that the higher the drug concentration, the more obvious the effect. There was no significant difference between the high-concentration and medium-concentration drug groups and the positive drug group.

**Table 10 Weight change of adipose tissue at administration site**

| Group | Mean wet adipose tissue weight (x̅±SEM, unit: g) | Mean wet adipose tissue weight/body weight percentage (x̅±SEM, %) |
|---|---|---|
| Blank control group | 0.370±0.029 | 0.93±0.10 |
| Positive control group | 0.224±0.022** | 0.57±0.05** |
| HXRZ-B high-dose group | 0.232±0.032* | 0.58±0.07* |
| HXRZ-B medium-dose group | 0.225±0.039* | 0.56±0.08* |
| HXRZ-B low-dose group | 0.326±0.106 | 0.76±0.21 |
| HXRZ-C high-dose group | 0.188±0.011** | 0.48±0.02** |
| HXRZ-C medium-dose group | 0.209±0.021** | 0.53±0.05* |
| HXRZ-C low-dose group | 0.198±0.030** | 0.51±0.08* |

| | | |
|---|---|---|
| Note: * represents comparison between the test sample and the blank control group, *p < 0.05, **p < 0.01. | | |

The adipose tissue collected from the administration site was prepared into pathological sections and subjected to H-E staining. The results are shown in FIG. 13. In each administration group, adipocyte degradation, lipid lake formation, immune cell infiltration and neogenic connective tissue formation were observed.

Further statistical analysis was performed on adipocyte density and adipocyte area. The adipose tissue from the inoculated side was immersed in adipose tissue fixative and sent for hematoxylin-eosin (H-E) staining. After imaging was completed, all measurements were standardized in millimeters. The area of adipose tissue in each image was measured, and the number of adipocytes in each image was counted. The area of a single adipocyte was calculated as: adipocyte area = total adipose tissue area/adipocyte number; adipocyte density = adipocyte number/total adipose tissue area. The statistical results are shown in Table 11. After drug intervention, adipocytes in the adipose tissue were damaged with cell enlargement and decreased cell density. Following four administrations, the adipose tissue at the administration sites was remodeled to form new connective tissue. The higher drug concentration indicated the more obvious effect. The effect of HXRZ-C was slightly better than that of HXRZ-B, which was similar to the adipose tissue wet weight results.

**Table 11 Changes in mean adipocyte density and mean adipocyte area of pathological sections in each group**

| Group | Mean adipocyte density (x̅±SEM, n/mm²) | Mean adipocyte area (x̅±SEM, mm²) |
|---|---|---|
| Blank control group | 997.7294±147.9755 | 0.001118±0.000190 |
| Positive control group | 322.3211±55.3047* | 0.003751±0.000614 |
| HXRZ-B high-dose group | 340.3195±31.7463* | 0.003177±0.000284 |
| HXRZ-B medium-dose group | 313.2046±85.5687* | 0.003554±0.000470 |
| HXRZ-B low-dose group | 418.7156±85.9688 | 0.002840±0.000267 |
| HXRZ-C high-dose group | 251.3400±31.0087* | 0.004632±0.000253 |
| HXRZ-C medium-dose group | 328.9026±63.1322* | 0.003297±0.000274 |
| HXRZ-C low-dose group | 355.3684±57.4571* | 0.003222±0.000161 |

| | | |
|---|---|---|
| Note: *represents comparison between the test sample and the blank control group, *P<0.05, **P<0.01. | | |

The above descriptions are merely embodiments of the present invention, but not intended to limit the present invention. For those skilled in the art, various variations and changes can be made to the present invention. Any amendments, equivalent replacements, improvements and the like made within the spirit and principle of the present invention should fall within the scope of appended claims.

## Claims

1. Use of a lipopeptide in the preparation of a fat-dissolving product.

2. The use according to claim 1, wherein the lipopeptide is a cyclic lipopeptide; optionally, the lipopeptide is one or more selected from surfactin, iturin, fengycin, daptomycin, polymyxin, bacillomycin L, plipastatin and bacillomycin D; more optionally, the lipopeptide is surfactin and/or daptomycin; more optionally, the lipopeptide is surfactin; more optionally, the surfactin is surfactin-C14 and/or surfactin-C15.

3. The use according to claim 1, wherein the fat-dissolving comprises lysing adipocytes and/or degrading adipose tissue.

4. A fat-dissolving product, containing a lipopeptide.

5. The fat-dissolving product according to claim 4, wherein the lipopeptide is a cyclic lipopeptide; optionally, the lipopeptide is one or more selected from surfactin, iturin, fengycin, daptomycin, polymyxin, bacillomycin L, plipastatin and bacillomycin D; more optionally, the lipopeptide is surfactin and/or daptomycin; more optionally, the lipopeptide is surfactin; more optionally, the surfactin is surfactin-C14 and/or surfactin-C15.

6. The fat-dissolving product according to claim 4, wherein by mass percentage, the content of the lipopeptide in the fat-dissolving product is 0.05%-3%; optionally, the content of the lipopeptide in the fat-dissolving product is 0.1%-1%.

7. A method for preparing the fat-dissolving product according to any one of claims 4-6, the method comprising: evenly dissolving a lipopeptide into a solvent.

8. The method according to claim 7, wherein the lipopeptide is a cyclic lipopeptide; optionally, the lipopeptide is one or more selected from surfactin, iturin, fengycin, daptomycin, polymyxin, bacillomycin L, plipastatin and bacillomycin D; more optionally, the lipopeptide is surfactin and/or daptomycin; more optionally, the lipopeptide is surfactin; more optionally, the surfactin is surfactin-C14 and/or surfactin-C15.

9. The method according to claim 7, wherein by mass percentage, the content of the lipopeptide is 0.05%-3%; more optionally, the content of the lipopeptide is 0.1%-1%.

10. The method according to claim 7, wherein the solvent is one or more selected from water, normal saline and phosphate buffer saline (PBS).
